# EUROPEAN PATENT SPECIFICATION

(11) **EP 3 000 823 B1**
(45) Date of publication and mention of the grant of the patent: **12.06.2019**
(21) Application number: 14791458.4
(22) Date of filing: 25.04.2014
(51) Int. Cl.: C07K 5/103, C07K 7/06, C07K 7/08, C07K 14/00, C12N 15/70, C12N 1/21, C12N 9/24, C12N 9/42, C12N 15/11

(54) **STRONG SECRETORY SIGNAL PEPTIDE ENHANCING SMALL PEPTIDE MOTIF AND USE THEREOF**
STARKES SEKRETORISCHES SIGNALPEPTID ZUR VERSTÄRKUNG VON KLEINEN PEPTIDMOTIVEN UND VERWENDUNG DAVON
PETIT MOTIF PEPTIDIQUE RENFORÇANT UN PEPTIDE SIGNAL DE SÉCRÉTION FORTE ET SON UTILISATION

(30) Priority: 03.05.2013 CN 201310162455
(43) Date of publication of application: 30.03.2016
(60) Divisional application: 17200981.3
(73) Proprietor: Nanjing University of Technology, Jiangsu 211816 (CN)
(72) Inventor: HE, Bingfang, Nanjing Jiangsu 211816 (CN); CHEN, Wenhua, Nanjing Jiangsu 211816 (CN); MI, Lan, Nanjing Jiangsu 211816 (CN); WU, Shanshan, Nanjing Jiangsu 211816 (CN); ZHU, Yun, Nanjing Jiangsu 211816 (CN); CHEN, Shanshan, Nanjing Jiangsu 211816 (CN)
(74) Representative: Proi World Intellectual Property GmbH
(86) International application number: PCT/CN2014/076249
(87) International publication number: WO 2014/177021

(56) References cited:
- EP-B1- 0 461 165
- WO-A2-02/057313
- CN-A- 103 254 277
- DATABASE UniProt [Online] 8 February 2011 (2011-02-08), "SubName: Full=Uncharacterized protein {ECO:0000313|EMBL:CBY13714.1};", XP002761884, retrieved from EBI accession no. UNIPROT:E4XVJ6 Database accession no. E4XVJ6
- SAGIYA, Y. ET AL.: 'Direct high-level secretion into the culture medium of tuna growth hormone in biologically active from by Bacillus brevis' APPLIED MICROBIOLOGY AND BIOTECHNOLOGY vol. 2 AND 3, no. 42, 30 November 1994, pages 358 - 363, XP035170965
- XIAO, JIE ET AL.: 'Advance in Improvement of Secretion Expression of Recombinant Protein Using E. coli' JOURNAL OF MICROBIOLOGY vol. 27, no. 2, 31 March 2007, pages 73 - 77, XP008181444

### SEQUENCE LISTING

<110> Nanjing University of Technology
<120> STRONG SECRETORY SIGNAL PEPTIDE ENHANCING SMALL PEPTIDE MOTIF AND USE THEREOF
<130> njut1304
<160> 96
<170> PatentIn version 3.3
<210> 1
   <211> 20
   <212> DNA
   <213> Artificial
<220>
   <223> P1
<400> 1
   gccaccgaac cagtgcctgg 20
<210> 2
   <211> 21
   <212> DNA
   <213> Artificial
<220>
   <223> P2
<400> 2
   ttactttgct actgctttgc c 21
<210> 3
   <211> 21
   <212> DNA
   <213> Artificial
<220>
   <223> P3
<400> 3
   atgaaaaaga cagctatcgc g 21
<210> 4
   <211> 35
   <212> DNA
   <213> Artificial
<220>
   <223> P4
<400> 4
   ctggttcggt ggcagcttgg gctacggtag cgaaa 35
<210> 5
   <211> 35
   <212> DNA
   <213> Artificial
<220>
   <223> P5
<400> 5
   accgtagccc aagctgccac cgaaccagtg cctgg 35
<210> 6
   <211> 30
   <212> DNA
   <213> Artificial
<220>
   <223> 6
<400> 6
   ccggaattct tactttgcta ctgctttgcc 30
<210> 7
   <211> 21
   <212> DNA
   <213> Artificial
<220>
   <223> P7
<400> 7
   atgaaatacc tattgcctac g 21
<210> 8
   <211> 35
   <212> DNA
   <213> Artificial
<220>
   <223> P8
<400> 8
   actggttcgg tggcagccat ggctggttgg gcagc 35
<210> 9
   <211> 35
   <212> DNA
   <213> Artificial
<220>
   <223> P9
<400> 9
   aaccagccat ggctgccacc gaaccagtgc ctggc 35
<210> 10
   <211> 30
   <212> DNA
   <213> Artificial
<220>
   <223> P10
<400> 10
   ccggaattct tactttgcta ctgctttgcc 30
<210> 11
   <211> 63
   <212> DNA
   <213> Artificial
<220>
   <223> OmpA
<400> 11
   atgaaaaaga cagctatcgc gattgcagtg gcactggctg gtttcgctac cgtagcccaa 60
   gct 63
<210> 12
   <211> 66
   <212> DNA
   <213> Artificial
<220>
   <223> PelB
<400> 12
   atgaaatacc tattgcctac ggcagccgct ggattgttat tactcgctgc ccaaccagcc 60
   atggct 66
<210> 13
   <211> 36
   <212> DNA
   <213> Artificial
<220>
   <223> P13
<400> 13
   cgccatatgg agagaatgaa aaagacagct atcgcg 36
<210> 14
   <211> 36
   <212> DNA
   <213> Artificial
<220>
   <223> P14
<400> 14
   cgccatatgg agagaatgaa atacctattg cctacg 36
<210> 15
   <211> 36
   <212> DNA
   <213> Artificial
<220>
   <223> P15
<400> 15
   cgccatatga gagagatgaa aaagacagct atcgcg 36
<210> 16
   <211> 36
   <212> DNA
   <213> Artificial
<220>
   <223> P16
<400> 16
   cgccatatga gagagatgaa atacctattg cctacg 36
<210> 17
   <211> 39
   <212> DNA
   <213> Artificial
<220>
   <223> P17
<400> 17
   cgccatatgg agagagcgat gaaaaagaca gctatcgcg 39
<210> 18
   <211> 39
   <212> DNA
   <213> Artificial
<220>
   <223> P18
<400> 18
   cgccatatgg agagagcgat gaaataccta ttgcctacg 39
<210> 19
   <211> 45
   <212> DNA
   <213> Artificial
<220>
   <223> P19
<400> 19
   cgccatatgg aggcgagagc ggcgatgaaa aagacagcta tcgcg 45
<210> 20
   <211> 45
   <212> DNA
   <213> Artificial
<220>
   <223> P20
<400> 20
   cgccatatgg aggcgagagc ggcgatgaaa tacctattgc ctacg 45
<210> 21
   <211> 48
   <212> DNA
   <213> Artificial
<220>
   <223> P21
<400> 21
   cgccatatgg cggcggagag agcgtgtatg aaaaagacag ctatcgcg 48
<210> 22
   <211> 48
   <212> DNA
   <213> Artificial
<220>
   <223> P22
<400> 22
   cgccatatgg cggcggagag agcgtgtatg aaatacctat tgcctacg 48
<210> 23
   <211> 42
   <212> DNA
   <213> Artificial
<220>
   <223> P23
<400> 23
   cgccatatga gagagattgt gatgaaaaag acagctatcg cg 42
<210> 24
   <211> 42
   <212> DNA
   <213> Artificial
<220>
   <223> P24
<400> 24
   cgccatatga gagagattgt gatgaaatac ctattgccta cg 42
<210> 25
   <211> 42
   <212> DNA
   <213> Artificial
<220>
   <223> P25
<400> 25
   cgccatatgg agagactctg tatgaaaaag acagctatcg cg 42
<210> 26
   <211> 42
   <212> DNA
   <213> Artificial
<220>
   <223> P26
<400> 26
   cgccatatgg agagactctg tatgaaatac ctattgccta cg 42
<210> 27
   <211> 51
   <212> DNA
   <213> Artificial
<220>
   <223> P27
<400> 27
   cgccatatga ccagaaccga ggcgtgtgcg atgaaaaaga cagctatcgc g 51
<210> 28
   <211> 51
   <212> DNA
   <213> Artificial
<220>
   <223> P28
<400> 28
   cgccatatga ccagaaccga ggcgtgtgcg atgaaatacc tattgcctac g 51
<210> 29
   <211> 54
   <212> DNA
   <213> Artificial
<220>
   <223> P29
<400> 29
   cgccatatgt gtagatgtga cgcgtgtgcg ctcatgaaaa agacagctat cgcg 54
<210> 30
   <211> 54
   <212> DNA
   <213> Artificial
<220>
   <223> P30
<400> 30
   cgccatatgt gtagatgtga cgcgtgtgcg ctcatgaaat acctattgcc tacg 54
<210> 31
   <211> 48
   <212> DNA
   <213> Artificial
<220>
   <223> P31
<400> 31
   cgccatatgg agagagcgtg cgcgctcatg aaaaagacag ctatcgcg 48
<210> 32
   <211> 48
   <212> DNA
   <213> Artificial
<220>
   <223> P32
<400> 32
   cgccatatgg agagagcgtg cgcgctcatg aaatacctat tgcctacg 48
<210> 33
   <211> 60
   <212> DNA
   <213> Artificial
<220>
   <223> P33
<400> 33
   cgccatatgg tggagctcac cagagcgtgc gcgctcgcga tgaaaaagac agctatcgcg 60
<210> 34
   <211> 60
   <212> DNA
   <213> Artificial
<220>
   <223> P34
<400> 34
   cgccatatgg tggagctcac cagagcgtgc gcgctcgcga tgaaatacct attgcctacg 60
<210> 35
   <211> 63
   <212> DNA
   <213> Artificial
<220>
   <223> P35
<400> 35
   cgccatatgg tggcggagct caccagagcg tgcgcgctcg cgatgaaaaa gacagctatc 60
   gcg 63
<210> 36
   <211> 63
   <212> DNA
   <213> Artificial
<220>
   <223> P36
<400> 36
   cgccatatgg tggcggagct caccagagcg tgcgcgctcg cgatgaaata cctattgcct 60
   acg 63
<210> 37
   <211> 51
   <212> DNA
   <213> Artificial
<220>
   <223> P37
<400> 37
   cgccatatgg agagagcgtg tgcgctcgcg atgaaaaaga cagctatcgc g 51
<210> 38
   <211> 51
   <212> DNA
   <213> Artificial
<220>
   <223> P38
<400> 38
   cgccatatgg agagagcgtg tgcgctcgcg atgaaatacc tattgcctac g 51
<210> 39
   <211> 57
   <212> DNA
   <213> Artificial
<220>
   <223> P39
<400> 39
   cgccatatgt gtctcgagag agcgtgcgcg ctcgcgatga aaaagacagc tatcgcg 57
<210> 40
   <211> 57
   <212> DNA
   <213> Artificial
<220>
   <223> P40
<400> 40
   cgccatatgt gtctcgagag agcgtgcgcg ctcgcgatga aatacctatt gcctacg 57
<210> 41
   <211> 63
   <212> DNA
   <213> Artificial
<220>
   <223> P41
<400> 41
   cgccatatgg tggcggagct caccagagcg tgcgcgctcg agatgaaaaa gacagctatc 60
   gcg 63
<210> 42
   <211> 63
   <212> DNA
   <213> Artificial
<220>
   <223> P42
<400> 42
   cgccatatgg tggcggagct caccagagcg tgcgcgctcg agatgaaata cctattgcct 60
   acg 63
<210> 43
   <211> 54
   <212> DNA
   <213> Artificial
<220>
   <223> P43
<400> 43
   cgccatatgg aggcgagagc gtgtgtggcg gtgatgaaaa agacagctat cgcg 54
<210> 44
   <211> 54
   <212> DNA
   <213> Artificial
<220>
   <223> P44
<400> 44
   cgccatatgg aggcgagagc gtgtgtggcg gtgatgaaat acctattgcc tacg 54
<210> 45
   <211> 51
   <212> DNA
   <213> Artificial
<220>
   <223> P45
<400> 45
   cgccatatgg agagagcgtg tgcgctcgcg atgaaaaaga cagctatcgc g 51
<210> 46
   <211> 51
   <212> DNA
   <213> Artificial
<220>
   <223> P46
<400> 46
   cgccatatgg agagagcgtg tgcgctcgcg atgaaatacc tattgcctac g 51
<210> 47
   <211> 54
   <212> DNA
   <213> Artificial
<220>
   <223> P47
<400> 47
   cgccatatgg acaaagcgtg ctgtgtggcg gtgatgaaaa agacagctat cgcg 54
<210> 48
   <211> 54
   <212> DNA
   <213> Artificial
<220>
   <223> P48
<400> 48
   cgccatatgg acaaagcgtg ctgtgtggcg gtgatgaaat acctattgcc tacg 54
<210> 49
   <211> 60
   <212> DNA
   <213> Artificial
<220>
   <223> P49
<400> 49
   cgccatatgc tcgacgtgag agcgtgtgcg ctcgcggcga tgaaaaagac agctatcgcg 60
<210> 50
   <211> 60
   <212> DNA
   <213> Artificial
<220>
   <223> P50
<400> 50
   cgccatatgc tcgacgtgag agcgtgtgcg ctcgcggcga tgaaatacct attgcctacg 60
<210> 51
   <211> 54
   <212> DNA
   <213> Artificial
<220>
   <223> P51
<400> 51
   cgccatatgg agagagcgtg cgcgctcgcg gcgatgaaaa agacagctat cgcg 54
<210> 52
   <211> 54
   <212> DNA
   <213> Artificial
<220>
   <223> P52
<400> 52
   cgccatatgg agagagcgtg cgcgctcgcg gcgatgaaat acctattgcc tacg 54
<210> 53
   <211> 69
   <212> DNA
   <213> Artificial
<220>
   <223> P53
<400> 53
   cgccatatgg cggcggacct caccaaagcg tgcgcgctcg cggcggcgat gaaaaagaca 60
   gctatcgcg 69
<210> 54
   <211> 69
   <212> DNA
   <213> Artificial
<220>
   <223> P54
<400> 54
   cgccatatgg cggcggacct caccaaagcg tgcgcgctcg cggcggcgat gaaataccta 60
   ttgcctacg 69
<210> 55
   <211> 57
   <212> DNA
   <213> Artificial
<220>
   <223> P55
<400> 55
   cgccatatgg agagagcgtg tgcgctcgcg gcggcgatga aaaagacagc tatcgcg 57
<210> 56
   <211> 57
   <212> DNA
   <213> Artificial
<220>
   <223> P56
<400> 56
   cgccatatgg agagagcgtg tgcgctcgcg gcggcgatga aatacctatt gcctacg 57
<210> 57
   <211> 60
   <212> DNA
   <213> Artificial
<220>
   <223> P57
<400> 57
   cgccatatgg agagagcgtg cgcgctcgcg gcggcggcga tgaaaaagac agctatcgcg 60
<210> 58
   <211> 60
   <212> DNA
   <213> Artificial
<220>
   <223> P58
<400> 58
   cgccatatgg agagagcgtg cgcgctcgcg gcggcggcga tgaaatacct attgcctacg 60
<210> 59
   <211> 75
   <212> DNA
   <213> Artificial
<220>
   <223> P59
<400> 59
   cgccatatga cctgcaaatg cctcgacgcg tgcgcgctcg cggcggcggc ggcgatgaaa 60
   aagacagcta tcgcg 75
<210> 60
   <211> 75
   <212> DNA
   <213> Artificial
<220>
   <223> P60
<400> 60
   cgccatatga cctgcaaatg cctcgacgcg tgcgcgctcg cggcggcggc ggcgatgaaa 60
   tacctattgc ctacg 75
<210> 61
   <211> 66
   <212> DNA
   <213> Artificial
<220>
   <223> P61
<400> 61
   cgccatatgg agagactcct ctgttgtacc accaccacca ccaccatgaa aaagacagct 60
   atcgcg 66
<210> 62
   <211> 66
   <212> DNA
   <213> Artificial
<220>
   <223> P62
<400> 62
   cgccatatgg agagactcct ctgttgtacc accaccacca ccaccatgaa atacctattg 60
   cctacg 66
<210> 63
   <211> 63
   <212> DNA
   <213> Artificial
<220>
   <223> P63
<400> 63
   cgccatatgg agagagcgtg cgcgctcgcg gcggcggcgg cgatgaaaaa gacagctatc 60
   gcg 63
<210> 64
   <211> 63
   <212> DNA
   <213> Artificial
<220>
   <223> P64
<400> 64
   cgccatatgg agagagcgtg cgcgctcgcg gcggcggcgg cgatgaaata cctattgcct 60
   acg 63
<210> 65
   <211> 75
   <212> DNA
   <213> Artificial
<220>
   <223> P65
<400> 65
   cgccatatgc tcaccaaatg cctcgagtgc gcgaccgcgt gttgttgttg ttgtatgaaa 60
aagacagcta tcgcg 75
<210> 66
   <211> 75
   <212> DNA
   <213> Artificial
<220>
   <223> P66
<400> 66
   cgccatatgc tcaccaaatg cctcgagtgc gcgaccgcgt gttgttgttg ttgtatgaaa 60
   tacctattgc ctacg 75
<210> 67
   <211> 63
   <212> DNA
   <213> Artificial
<220>
   <223> P67
<400> 67
   cgccatatgg agagaaccac cctcacctgc tgctgctgct gcatgaaaaa gacagctatc 60
   gcg 63
<210> 68
   <211> 63
   <212> DNA
   <213> Artificial
<220>
   <223> P68
<400> 68
   cgccatatgg agagaaccac cctcacctgc tgctgctgct gcatgaaata cctattgcct 60
   acg 63
<210> 69
   <211> 72
   <212> DNA
   <213> Artificial
<220>
   <223> P69
<400> 69
   cgccatatgg agagagcgtg cgtggcggtg gagagagcgt gcgtggcggt gatgaaaaag 60
   acagctatcg cg 72
<210> 70
   <211> 72
   <212> DNA
   <213> Artificial
<220>
   <223> P70
<400> 70
   cgccatatgg agagagcgtg cgtggcggtg gagagagcgt gcgtggcggt gatgaaatac 60
   ctattgccta cg 72
<210> 71
   <211> 72
   <212> DNA
   <213> Artificial
<220>
   <223> P71
<400> 71
   cgccatatgg agagagcgtg cgcgctcgcg gtggagagag cgtgcgcgct catgaaaaag 60
   acagctatcg cg 72
<210> 72
   <211> 72
   <212> DNA
   <213> Artificial
<220>
   <223> P72
<400> 72
   cgccatatgg agagagcgtg cgcgctcgcg gtggagagag cgtgcgcgct catgaaatac 60
   ctattgccta cg 72
<210> 73
   <211> 90
   <212> DNA
   <213> Artificial
<220>
   <223> P73
<400> 7
<210> 74
   <211> 90
   <212> DNA
   <213> Artificial
<220>
   <223> P74
<400> 74
   cgccatatgg agagagcgtg cgcgctcgtg gagagagcgt gcgcgctcgt ggagagagcg 60
tgtgcgctca tgaaatacct attgcctacg 90
<210> 75
   <211> 105
   <212> DNA
   <213> Artificial
<220>
   <223> P75
<400> 75
   cgccatatgg agagatgcct cgcgaccctc gtggagagac tctgcgtggc ggtggtggag 60
   agagcgtgcg cgtgcgcgct cgcgatgaaa aagacagcta tcgcg 105
<210> 76
   <211> 105
   <212> DNA
   <213> Artificial
<220>
   <223> P76
<400> 76
   cgccatatgg agagatgcct cgcgaccctc gtggagagac tctgcgtggc ggtggtggag 60
   agagcgtgcg cgtgcgcgct cgcgatgaaa tacctattgc ctacg 105
<210> 77
   <211> 30
   <212> DNA
   <213> Artificial
<220>
   <223> P77
<400> 77
   cgccatatga tgaaaaagac agctatcgcg 30
<210> 78
   <211> 30
   <212> DNA
   <213> Artificial
<220>
   <223> P78
<400> 78
   cgccatatga tgaaatacct attgcctacg 30
<210> 79
   <211> 21
   <212> DNA
   <213> Artificial
<220>
   <223> P79
<400> 79
   caaacaggtg gatcgttttt t 21
<210> 80
   <211> 24
   <212> DNA
   <213> Artificial
<220>
   <223> P80
<400> 80
   ttattttttt gtatagcgca ccca 24
<210> 81
   <211> 21
   <212> DNA
   <213> Artificial
<220>
   <223> P81
<400> 81
   atgaaaaaga cagctatcgc g 21
<210> 82
   <211> 36
   <212> DNA
   <213> Artificial
<220>
   <223> P82
<400> 82
   aaaaaacgat ccacctgttt gagcttgggc tacggt 36
<210> 83
   <211> 37
   <212> DNA
   <213> Artificial
<220>
   <223> P83
<400> 83
   taccgtagcc caagctcaaa caggtggatc gtttttt 37
<210> 84
   <211> 32
   <212> DNA
   <213> Artificial
<220>
   <223> P84
<400> 84
   ccggaatttt atttttttgt atagcgcacc ca 32
<210> 85
   <211> 21
   <212> DNA
   <213> Artificial
<220>
   <223> P85
<400> 85
   atgaaatacc tattgcctac g 21
<210> 86
   <211> 42
   <212> DNA
   <213> Artificial
<220>
   <223> P86
<400> 86
   aaaaaacgat ccacctgttt gagccatggc tggttgggca gc 42
<210> 87
   <211> 37
   <212> DNA
   <213> Artificial
<220>
   <223> P87
<400> 87
   ccaaccagcc atggctcaaa caggtggatc gtttttt 37
<210> 88
   <211> 32
   <212> DNA
   <213> Artificial
<220>
   <223> P88
<400> 88
   ccggaatttt atttttttgt atagcgcacc ca 32
<210> 89
   <211> 48
   <212> DNA
   <213> Artificial
<220>
   <223> P89
<400> 89
   cgccatatgg aacgagcatg tgttgcaatg aaaaagacag ctatcgcg 48
<210> 90
   <211> 48
   <212> DNA
   <213> Artificial
<220>
   <223> P90
<400> 90
   cgccatatgg aacgagcatg tgttgcaatg aaatacctat tgcctacg 48
<210> 91
   <211> 78
   <212> DNA
   <213> Artificial
<220>
   <223> P91
<400> 91
   cgccatatgg tggagagact atgtgtggca gtggttgaaa gggcgtgtgc gctagcgatg 60
   aaaaagacag ctatcgcg 78
<210> 92
   <211> 78
   <212> DNA
   <213> Artificial
<220>
   <223> P92
<400> 92
   cgccatatgg tggagagact atgtgtggca gtggttgaaa gggcgtgtgc gctagcgatg 60
   aaatacctat tgcctacg 78
<210> 93
   <211> 102
   <212> DNA
   <213> Artificial
<220>
   <223> P93
<400> 93
   cgccatatgg ttgaaaggtg tctcgcgacc ctcgtggaga gactatgtgt ggcagtggtt 60
   gaaagggcgt gtgcgctagc gatgaaaaag acagctatcg cg 102
<210> 94
   <211> 102
   <212> DNA
   <213> Artificial
<220>
   <223> P94
<400> 94
   cgccatatgg ttgaaaggtg tctcgcgacc ctcgtggaga gactatgtgt ggcagtggtt 60
gaaagggcgt gtgcgctagc gatgaaatac ctattgccta cg 102
<210> 95
   <211> 30
   <212> **DNA**
   <213> Artificial
<220>
   <223> P95
<400> 95
   cgccatatga tgaaaaagac agctatcgcg 30
<210> 96
   <211> 30
   <212> DNA
   <213> Artificial
<220>
   <223> P96
<400> 96
   cgccatatga tgaaatacct attgcctacg 30

## Description

### Technical Field

The present invention belongs to the fields of protein engineering and genetic engineering, in particular, the present invention relates to small peptides, enhancing the secretion of signal peptides, and the use thereof.

### Description of the Related Art

The secretion system of proteins is not only the premise of protein sorting and protein positioning, as well as the realization of physiological function, but also an important means for genetic engineering and protein engineering. People have worked out various prokaryotic and eukaryotic expression systems to produce recombinant proteins. The E. coli expression system has attracted much attention due to its clear genetic background, its simple operation, its rapid growth in cheap culture medium, and its availability for large-scale fermentation and culturing, etc. The E. coli system has been used for preparing a variety of enzymatic preparations at present, even for preparing some pharmaceutical proteins including interferon, insulin, human serum albumin, etc. and, thus, has made remarkable achievements in the biotechnology field.

In principle, recombinant proteins can be produced in E.coli by the following modes: 1. by intracellular production of soluble proteins; 2. by intracellular production of inclusion bodies; 3. by secretion to the periplasmic space or the extracellular medium. The E. coli system is applicable to the proteins which shall not be modified after production and translation. A large amount of intermediates are often accumulated to form settlements of inclusion bodies due to the absence of cofactors, required in the protein folding process of E. coli. Refolding and other complex processes are also required, and the periplasmic oxidized redox environment is conductive to protein folding. If the proteins are located in the periplasmic space after crossing the intracellular membrane, even directly secreted to the extracellular medium after passing through the epicyte, the downstream refolding, separation and purification processes can be greatly simplified to reduce toxicity and metabolic burden of proteins on host bacteria and this significantly improves the expression quantity of recombinant proteins.

Most existing secretory expression systems secret into the periplasmic space, and one still needs to obtain the recombinant proteins by disrupting and purifying the cells. A small amount of systems secret to the extracellular space, yet this will cause very insufficient secretion efficiency and also post-processing problems of fusion proteins due to incomplete protein secretion system of E. coli itself, or cognitive limitation on the secretion system.

Signal peptides are polypeptides, containing about 10-60 amino acids, and are generally located at the N-end of secreted proteins. Using the signal peptides for the secretory expression of proteins, the general operating method is to clone the encoding gene of a target protein to the back end of the signal peptide sequence, realize the fusion of the target gene and the signal peptide sequence at the transcriptional and translational level in a host, and guide the combination of the whole polypeptide chain, as well as intracellular molecular chaperone, or secretion signal recognition particles, membrane positioning or secretion, to the extracellular space by the signal peptides at the N-end of the protein precursor.

At present, the E. coli system has many restrictions on extensive application of the strategy, though the extracellular secretory expression strategy has more obvious advantages in comparison with other expression modes. Thus, the prerequisite is to possess a signal peptide, capable of carrying and secreting the target proteins to the extracellular space. Even if a signal peptide has the ability to mediate the extracellular protein secretion, it does not indicate that a specific signal peptide has for all recombinant proteins the ability to mediate this extracellular protein secretion or has the ability of the same level. Each recombinant protein has its own specific encoding gene, its transition points of the signal peptides, and the recombinant proteins have different sequences, and the structures of the recombinant proteins are different, therefore, the secretion level of the recombinant proteins depends on the optimization level of the signal peptides, the transmembrane structure, favorable to the target proteins and the matching level there between.

In the E. coli expression system, the basic secretion pathway of secreted proteins crossing the plasma membrane is based on the Sec or on the Tat system, e.g., the outer membrane protein (OmpA) signal peptide and the pectate lyase (PelB) signal peptide are both secreted by Sec, the trimethylamine N-oxide reductase (TorA) signal peptide is secreted by Tat. The two types of signal peptides have been successfully used in the case for the secretory expression of exogenous proteins.

With regard to incorrect or incomplete hydrolysis processing of target proteins in the yeast processing system, Patent EP 0461165 B1 has disclosed a polypeptide structure, fused with hydrophobic signal peptides, with hydrophilic leading peptides and with heterologous proteins. The amino acid sequence between the C-end of the leading peptide and / or the N-end of the exogenous protein (near the yeast processing site) is modified to have negatively charged amino acids near the yeast processing site and to facilitate the full exposure of the processing site. Thereby improving the shearing efficiency of the protease, increasing the expression quantity of correctly processed (activated) target proteins, and then secreting the target proteins to the extracellular space. Database entry UNIPROT E4XVJ6 discloses an "uncharacterized protein" of 605 amino acids comprising MERACVA, i.e. a seven-amino acid overlap with MERACVAV. The document remains silent about secretion.

### Summary of the Invention

One technical purpose of the present invention is to provide small peptides having the function of enhancing signal peptide secretion, being fused to the front end of an ompA signal peptide or a pelB signal peptide, so as to achieve the ability of the above signal peptides for the secretory expression of exogenous proteins in the E. coli system.

The other technical purpose of the present invention is to provide the use of the small peptides, having the function of enhancing signal peptide secretion, i.e., a method by using the small peptides for constructing a vector for enhancing the secretion ability of common signal peptides to improve the method for the secretory expression of exogenous proteins.

In the present invention, the term "secretion" means that protein or peptide molecules are transported to the outside of the bacterial cell, but it also includes the cases: where protein or peptide molecules finally stand in the culture medium in completely free form, and some proteins are out of the bacteria or exist in the periplasmic space of the bacteria.

The following terms, used in the present invention, have the following meanings, unless otherwise specified:
A "variant" of a protein amino acid or a polynucleotide refers to an amino acid sequence out of changes of one or more amino acid(s) or nucleotide(s) or the polynucleotide sequence for encoding amino acids or nucleotides. The said "changes" may comprise a deletion, an insertion or a replacement of amino acids or nucleotides in the amino acid sequence or in the nucleotide sequence. The variant may have conservative changes, the replaced amino acids have the structure or chemical properties similar to the original amino acids, for example, isoleucine is replaced with leucine. The variant also may have non-conservative changes, for example, glycine is replaced with tryptophan.
"Deletion" refers to the deletion of one or more amino acid(s) or nucleotide(s) in the amino acid sequence or in the nucleotide sequence. "Insertion" or "Addition" refers to the addition of one or more amino acid(s) or nucleotide(s) due to changes in the amino acid sequence or in the nucleotide sequence, compared to the original molecules. "Replacement" means that one or more amino acid(s) or nucleotide(s) are replaced with different amino acids or nucleotides.

In order to achieve the technical purpose of the present invention, the technical scheme is as follows:
Small peptides, characterized by comprising an amino acid sequence having the following formula: M(αXβYγ/αYβXγ)ₙ,
Wherein X represent an acidic amino acid;
Y represents an alkaline amino acid;
α is 0 to 2 neutral amino acid(s);
β represents 0 to 2 neutral amino acid(s);
γ represents 1 to 10 neutral amino acid(s);
n is 1 to 3.

Where, "/" in the formula means "or'.

Further, the acidic amino acid represented by X is preferably Glu or Asp.

Further, the basic amino acid represented by Y is preferably Arg or Lys.

Further, the neutral amino acid is preferably Ala, Cys, Leu, Val, Ile or Phe.

Further, when n=1, the small peptide motifs of the present invention have optimal enhancing secretion effect.

Further, as a most preferred embodiment of the present invention, the small peptides have optimal enhancing secretion effect when α is 1 neutral amino acid, β is 0 neutral amino acid, γ is 2-5 neutral amino acids, X is Glu and Y is Arg.

Therefore, with regard to the small peptides protected by the present invention and obtained by the above formula, the original small peptide is MERACVAV, and the derived analogues can change into:
MREACVAV
MAERACVAV;
MEARACVAV;
MDKACVAV;
MERLIVFAV;....

Or overlaps of small peptides , e.g., MERACVA+VEARLIVFAV.

In one embodiment, the present invention relates to a small peptide, characterized by a) enhancing signal peptide secretion, b) being fused to the front end of a signal peptide, and c) having an amino acid MERACVAV.

In another embodiment of the invention, said small peptide is further characterized by being suitable for inducible expression.

In still another embodiment of the invention, said small peptide is additionally characterized by being fused to the N-end of an ompA or of a pelB signal peptide.

In yet another embodiment of the invention, said small peptide is further characterized by being suitable for use in E. coli.

In a further embodiment, the invention relates to a polynucleotide encoding said small peptide.

In yet another embodiment, the invention relates to a recombinant vector comprising said polynucleotide.

In still a further embodiment of the invention said recombinant vector is a plasmid.

In another embodiment, the invention relates to a host cell comprising said recombinant vector by means of transformation or transfection.

In yet a further embodiment, the invention relates to the use of said small peptide, for constructing a vector, enhancing the secretion ability of signal peptides to improve the secretory expression of exogenous proteins.

Specifically, it means that the small peptides of the present invention are fused to the front end of signal peptides, the constructed secretion vector is transformed into the E. coli expression host for inducible expression to achieve the secretion enhancing function of the small peptides.

In one Embodiment of the present invention, fructosidase FRU6 from *Arthrobacter arilaitensis* and dextranase BGL from *B.subtilis* are taken as target proteins, the common signal peptides are selected from ompA of outer membrane protein and from pelB signal peptide of pectate lyase. After the small peptides of the present invention are fused to the front end of ompA or pelB signal peptides, the constructed secretion vector is transformed into the E. coli expression host for inducible expression to then verify the secretion enhancing function of the small peptide by the testing method.

The host adopted by the present invention is *E.coil BL21*(DE3), which is a basic framework, constructed by taking E. coli pET-22b as a vector to remove pelB signal peptide sequence in the original pET-22b. The ompA signal peptide / pelB signal peptide and fructosidase FRU6 or dextranase BGL are fused by overlapping PCR, and the upstream primers are designed to add small peptide genes before the signal peptide gene sequence. Secretion enhancing expression vectors pET-EompA-FRU6, pET-EpeIB-FRU6, pET-EompA-BGL and pET-EpelB-BGL are constructed by enzyme cutting and connection at the enzyme cutting site.

The recombinant plasmid vectors pET-EompA-FRU6 and pET-EpelB-FRU6 (or pET-EompA-BGL and pET-EpelB-BGL) contain fused secretion enhancing signal peptides and fructosidase FRU6 (or dextranase BGL), and are respectively named as BL21/ pET-EompA-FRU6 and as BL21/pET-EpelB-FRU6 (or BL21/pET-EompA-BGL and BL21/pET-EpelB-BGL) after being transformed into the host E.coli BL21. The inducer is IPTG when the LB culture medium is subject to inducible expression.

The results obtained after the secretory expression of the above two target proteins are acceptable, when testing the enzyme activities of intracellular and extracellular culture media and analyses on SDS-PAGE.

The secretion examples adopted by the present invention include fructosidase FRU6 and β-1,3-1,4 dextranase BGL, Fructosidase FRU6 (molecular weight is about 55kDa from *Arthrobacter arilaitensis* NJEM01, strain Collection No. CCTCC M 2012155). The classification No. of the fructosidase system is EC 3.2.1.80, which is an extracellular enzyme, so the 2,1-β-glucosidic bond or the 2,6-β-glucosidic bond at the non-reducing end of the fructosan molecule composed of the β-D-fructose can be specifically catalyzed and hydrolyzed, in addition, synanthrin, sucrose, raffinose, etc. can also be hydrolyzed. Such extracellular enzymes have high utilization values in the biological, in the medical, and in the food fields. Dextranase BGL (molecular weight is about 28 kDa, EC 3.2.1.73, β-dextranase is from B. subtilis) is an incision hydrolase, which can efficiently and metastatically hydrolyze β-1,4 glucosidic bond close to β-1,3 glucosidic bond in β-glucan, thereby reducing adverse impact of β-glucan in cereals on industrial production. It also has very important application value in the beer brewing industry, in the feed industry and in other fields.

In addition, the beneficial effects of the present invention are as follows: the small peptides, capable of enhancing the secretion function are connected with the N-end of the signal peptide and have obvious secretion enhancing effects on target proteins. The test on the secretory expression example of fructosidase FRU6 shows, that the secretion efficiency is increased by 5.1 times to the utmost extent after addition of a small peptide before the ompA signal peptide. The secretion efficiency is increased by 5.4 times to the utmost extent after addition of a small peptide before the pelB signal peptide. The test on the secretory expression example of dextranase BGL shows that the secretion efficiency of the small peptides is increased by 2.3 times, compared to that of ompA signal peptide, and 2.5 times compared to that of PelB signal peptide.

With regard to the polypeptide structure disclosed in EP 0461165 B1, it is to fully expose the yeast processing site by modifying the amino acid sequence between the C-end of the leading peptide and / or the N-end of the exogenous protein, thereby promoting the correct processing of the target proteins and improving the active protein expression quantity. In addition, the secretory expression vector constructed in the present invention can be used to produce a variety of recombinant proteins.

### Brief Description of the Drawings

Figure 1 is a map of the recombinant vector pET-EompA-FRU6;
   Wherein ompA SP is the encoding sequence of the signal peptide of the outer membrane protein A; FRU6 is the encoding sequence of fructosidase FRU6; Amp is the encoding sequence of the resistant gene ampicillin, and T7 is the promoter.
Figure 2 is the relationship between the secretion enhancing efficiency of small peptides and the fermentation time.
Figure 3 is the comparison of the activity between BL21/pET-EompA-FRU6 and BL21/pET-EpelB-FRU6 in the culture medium and the negative control fermenting supernatant enzyme.
Figure 4 is an electrophoretogram of an SDS-PAGE of BL21/pET-EompA-FRU6 fermentation liquor in the culture medium;
   Wherein M is the marker of protein; 1 and 2 are the negative controls for the BL21 strain fermenting supernatant and thallus fragmentized supernatant without a signal peptide sequence added before fructosidase FRU6; 3 and 4 are BL21/pET-ompA-FRU6 strain fermenting supernatant and thallus fragmentized supernatant. 5 and 6 are BL21/pET-EompA-FRU6 strain fermenting supernatant and thallus fragmentized supernatant. (In the pET-EompA-FRU6 plasmid, the small peptide sequence added before the ompA signal peptide is MERACALA). The arrow direction is the molecular weight position for the mature peptide of fructosidase FRU6.

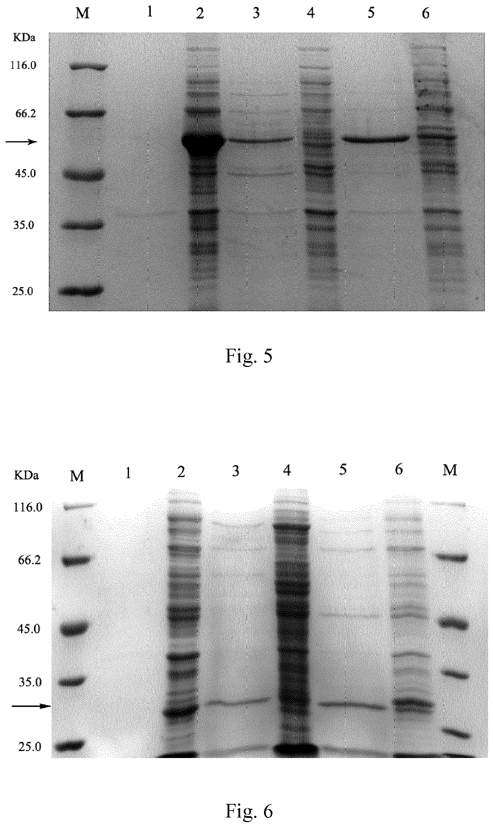

Figure 5 is an electropherogram of an SDS-PAGE of BL21/pET-EpelB-FRU6 fermentation liquor in LB culture medium.
   Wherein M is the marker of protein; 1 and 2 are the negative controls for the BL21 strain fermenting supernatant and the thallus fragmentized supernatant without a signal peptide sequence added before fructosidase FRU6; 3 and 4 are BL21/pET-pelB-FRU6 strain fermenting supernatant and thallus fragmentized supernatant. 5 and 6 are BL21/pET-EpelB-FRU6 strain fermenting supernatant and thallus fragmentized supernatant. (In pET-EpelB-FRU6 plasmid, the small peptide sequence added before the pelB signal peptide is MERACALA). The arrow direction is the molecular weight position for the mature peptide of fructosidase FRU6.
Figure 6 is an electrophoretogram of SDS-PAGE of BL21/pET-EompA-BGL fermentation liquor in the culture medium;
   Wherein M is the marker of protein; 1 and 2 are the negative controls for the BL21 strain fermenting supernatant and the thallus fragmentized supernatant without a signal peptide sequence added before dextranase BGL; 3 and 4 are the BL21/pET-ompA-BGL strain fermenting supernatant and the thallus fragmentized supernatant. 5 and 6 are BL21/pET-EompA-BGL strain fermenting supernatant and thallus fragmentized supernatant. (In pET-EompA-BGL plasmid, the small peptide sequence added before the ompA signal peptide is MERACALA). The arrow direction is the molecular weight position for the mature peptide of dextranase BGL.

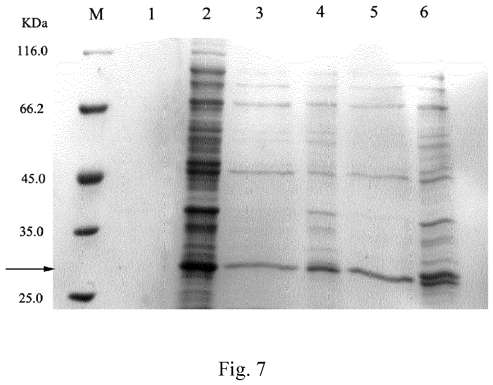

Figure 7 is an electropherogram of SDS-PAGE of BL21/pET-EpelB-BGL fermentation liquor in LB culture medium.
   Wherein M is the marker of protein; 1 and 2 are the negative controls for the BL21 strain fermenting supernatant and the thallus fragmentized supernatant without a signal peptide sequence added before dextranase BGL; 3 and 4 are the BL21/pET-pelB-BGL strain fermenting supernatant and the thallus fragmentized supernatant; 5 and 6 are BL21/pET-EpelB-BGL strain fermenting supernatant and thallus fragmentized supernatant. (In pET-EpelB-BGL plasmid, the small peptide sequence added before the pelB signal peptide is MERACALA). The arrow direction is the molecular weight position for the mature peptide of dextranase BGL.

### Embodiments

Unless otherwise specified, the methods of the following embodiments are conventional methods, and the involved plasmids, reagents and other materials can be commercially obtained.

### Embodiment 1: Use of small peptides for secretory expression of fructosidase

First, the fructosidase Fru6 gene in this embodiment is from the *Arthrobacter arilaitensis* NJEM01 strain, which was previously applied by the inventor in China, the patent application No. is CN102732456A and the Collection No. of the strain is CCTCC NO: M 2012155.

All primers involved in this embodiment are synthesized by the company Invitrogen (see table 1). The following primers are uniformly expressed as "P" plus the No., for example, for the No. 45 primer in table 2, the code is P45 and the corresponding No. in the sequence table is SEQ ID NO: 45.

**Table 1 Primers required for Embodiment 1 and the nucleotide sequence of the synthetic sequence**

| SEQ ID NO | **Nucleotide sequence of primer (5'-3')** |
|---|---|
| **1** | **GCCACCGAACCAGTGCCTGG** |
| **2** | **TTACTTTGCTACTGCTTTGCC** |
| **3** | **ATGAAAAAGACAGCTATCGCG** |
| **4** | |
| **5** | |
| **6** | |
| **7** | **ATGAAATACCTATTGCCTACG** |
| **8** | |
| **9** | |
| **10** | |

| | |
|---|---|
| Note: the underlined part in the table is shown as the restriction enzyme cutting site. | |

(1) Obtaining the fructosidase FRU6 gene: taking the genome of the *Arthrobacter arilaitensis* NJEM01 strain as a template, the primers P1 and P2 are used for PCR reaction so as to amplify the gene segment of fructosidase FRU6 (excluding the signal peptide sequence of the enzyme itself), the segment is cloned into the clone vector pMD18-T to obtain pMD-T-FRU6, and transformed into the clone host DH5α. The testing on the DNA sequence can validate the correctness.
(2) Fusion of signal peptide and fructosidase gene: The ompA and pelB signal peptide gene sequences (i.e., primer P11 and primer P12) are artificially synthesized to extract the plasmid pMD-T-FRU6 from step (1). PCR primers P3-P6 or P7-P10 are overlapped after being designed, fructosidase FRU6 and signal peptide ompA or pelB are fused respectively. P6 and P10 all have *EcoR*□enzyme cutting sites.
(3) Design of secretion enhancing vector: The amino acid sequence of the small peptide is MERACALA. See table 2 and table 3 for more small peptides.

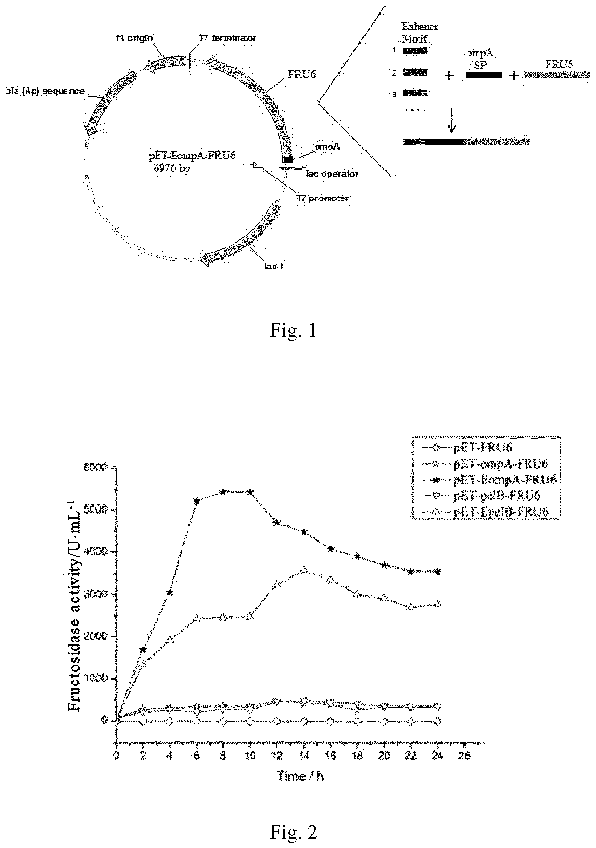

The fusion gene in step (2) is taken as a template, and the upstream primers P45 and P46 have a *Nde* I enzyme cutting site after being designed. Upon common PCR reaction, primers P45 and P6 are combined to add the small peptide gene before the ompA signal peptide gene; primers P46 and P10 are combined to add the small peptide gene (e.g., the amino acid sequence of the small peptide is MERACALA) before the pelB signal peptide gene. A *Nde* I and *EcoR* I double-enzyme digestion is used to obtain the target segments at the cohesive end. Vector pET-22b (purchased from the company Invitrogen) is subjected to enzyme cutting degradation by the restriction enzymes *Nde* I and *EcoR* I, to recover and remove large plasmid segments of the pelB signal peptide sequence. Then the recovered large plasmid segments and the enzyme cutting product of the secretion enhancing signal peptide and the fructosidase fusion gene are connected by T4 ligase to obtain pET-EompA-FRU6 (see figure 1 for the plasmid map) and pET-EpelB-FRU6 (neglected when the plasmid map is similar to the former map) and transformed into the clone host E. coli DH5α, so the testing on the DNA sequence can validate the correctness.

**Table 2 Amino acid sequences, primers and enzyme activities of small peptides involved in corresponding fructosidase**

| **n** | **M** | **α** | **X (or Y)** | **β** | **Y (or X)** | **γ** | **Primer** | **Signal peptide** | **Enzyme activity** |
|---|---|---|---|---|---|---|---|---|---|
| 1 | M | | E | | R | | 13 | ompA | 1279 |
| | | | | | | | 14 | pelB | 1521 |
| 1 | M | | R | | E | | 15 | ompA | 801 |
| | | | | | | | 16 | pelB | 917 |
| 1 | M | | E | | R | A | 17 | ompA | 1831 |
| | | | | | | | 18 | pelB | 1734 |
| 1 | M | | E | A | R | AA | 19 | ompA | 1678 |
| | | | | | | | 20 | pelB | 1530 |
| 1 | M | AA | E | | R | AC | 21 | ompA | 2169 |
| | | | | | | | 22 | pelB | 1781 |
| 1 | M | | R | | E | IV | 23 | ompA | 907 |
| | | | | | | | 24 | pelB | 862 |
| 1 | M | | E | | R | LC | 25 | ompA | 2380 |
| | | | | | | | 26 | pelB | 1972 |
| 1 | M | T | R | T | E | ACA | 27 | ompA | 2024 |
| | | | | | | | 28 | pelB | 1976 |
| 1 | M | C | R | C | D | ACAL | 29 | ompA | 2175 |
| | | | | | | | 30 | pelB | 2005 |
| 1 | M | | E | | R | ACAL | 31 | ompA | 2169 |
| | | | | | | | 32 | pelB | 1781 |
| 1 | M | V | E | LT | R | ACALA | 33 | ompA | 2513 |
| | | | | | | | 34 | pelB | 2100 |
| 1 | M | | E | | R | ACALA | 35 | ompA | 1877 |
| | | | | | | | 36 | pelB | 1762 |
| 1 | M | CL | E | | R | ACALA | 37 | ompA | 2395 |
| | | | | | | | 38 | pelB | 2230 |
| 1 | M | V | E | T | R | ACALA | 39 | ompA | 2460 |
| | | | | | | | 40 | pelB | 2195 |
| 1 | M | VA | E | LT | R | ACALA | 41 | ompA | 2380 |
| | | | | | | | 42 | pelB | 2120 |
| 1 | M | | E | A | R | ACVAV | 43 | ompA | 2390 |
| | | | | | | | 44 | pelB | 2275 |
| 1 | M | | E | | R | ACALA | 45 | ompA | 2016 |
| | | | | | | | 46 | pelB | 1979 |
| | M | | D | | K | ACVAV | 47 | ompA | 1193 |
| | | | | | | | 48 | pelB | 1272 |
| 1 | M | L | D | V | R | ACALAA | 49 | ompA | 1754 |
| | | | | | | | 50 | pelB | 1644 |
| 1 | M | | E | | R | ACALAA | 51 | ompA | 1980 |
| | | | | | | | 52 | pelB | 1642 |
| 1 | M | AA | D | LT | K | | 53 | ompA | 1766 |
| | | | | | | | 54 | pelB | 1790 |
| 1 | M | | E | | R | | 55 | ompA | 1987 |
| | | | | | | | 56 | pelB | 1755 |
| 1 | M | | E | | R | | 57 | ompA | 2485 |
| | | | | | | | 58 | 1pelB | 1643 |
| 1 | M | TC | K | CL | D | | 59 | ompA | 1906 |
| | | | | | | | 60 | pelB | 1560 |
| 1 | M | | E | | R | | 61 | ompA | 1986 |
| | | | | | | | 62 | pelB | 1756 |
| 1 | M | | E | | R | | 63 | o1mpA | 1762 |
| | | | | | | | 64 | pelB | 1882 |
| 1 | M | LT | K | CL | E | | 65 | ompA | 1883 |
| | | | | | | | 66 | pelB | 1986 |
| 1 | M | | E | | R | | 67 | ompA | 1880 |
| | | | | | | | 68 | pelB | 1670 |
| 2 | MERACVAV+MERACVAV | | | | | | 69 | ompA | 1787 |
| | | | | | | | 70 | pelB | 1754 |
| 2 | MERACALA+VERACAL | | | | | | 71 | ompA | 2460 |
| | | | | | | | 72 | pelB | 1845 |
| 3 | MERACAL+VERACAL+VERACAL | | | | | | 73 | ompA | 1680 |
| | | | | | | | 74 | pelB | 1855 |
| 3 | MERCLATL+VERLCVAV+VERACALA | | | | | | 75 | ompA | 2260 |
| | | | | | | | 76 | pelB | 2042 |
| 0 | Blank | | | | | | 77 | ompA | 420 |
| | | | | | | | 78 | pelB | 495 |

| | | | | | | | | | |
|---|---|---|---|---|---|---|---|---|---|
| Note: the primer No. in the table corresponds to P or SEQ ID NO. | | | | | | | | | |

OmpA and PelB signal peptide sequences are as follows:
OmpA:
PelB:

**Table 3 Upstream primers correspondingly designed for the small peptides of Table 2**

| Primer | Sequence (5'→3') |
|---|---|
| 13 | CGCCATATGGAGAGAATGAAAAAGACAGCTATCGCG |
| 14 | CGCCATATGGAGAGAATGAAATACCTATTGCCTACG |
| 15 | CGCCATATGAGAGAGATGAAAAAGACAGCTATCGCG |
| 16 | CGCCATATGAGAGAGATGAAATACCTATTGCCTACG |
| 17 | CGCCATATGGAGAGAGCGATGAAAAAGACAGCTATCGCG |
| 18 | CGCCATATGGAGAGAGCGATGAAATACCTATTGCCTACG |
| 19 | CGCCATATGGAGGCGAGAGCGGCGATGAAAAAGACAGCTATCGCG |
| 20 | CGCCATATGGAGGCGAGAGCGGCGATGAAATACCTATTGCCTACG |
| 21 | CGCCATATGGCGGCGGAGAGAGCGTGTATGAAAAAGACAGCTATCGCG |
| 22 | CGCCATATGGCGGCGGAGAGAGCGTGTATGAAATACCTATTGCCTACG |
| 23 | CGCCATATGAGAGAGATTGTGATGAAAAAGACAGCTATCGCG |
| 24 | CGCCATATGAGAGAGATTGTGATGAAATACCTATTGCCTACG |
| 25 | CGCCATATGGAGAGACTCTGTATGAAAAAGACAGCTATCGCG |
| 26 | CGCCATATGGAGAGACTCTGTATGAAATACCTATTGCCTACG |
| 27 | CGCCATATGACCAGAACCGAGGCGTGTGCGATGAAAAAGACAGCTATCGCG |
| 28 | CGCCATATGACCAGAACCGAGGCGTGTGCGATGAAATACCTATTGCCTACG |
| 29 | CGCCATATGTGTAGATGTGACGCGTGTGCGCTCATGAAAAAGACAGCTATCGCG |
| 30 | CGCCATATGTGTAGATGTGACGCGTGTGCGCTCATGAAATACCTATTGCCTACG |
| 31 | CGCCATATGGAGAGAGCGTGCGCGCTCATGAAAAAGACAGCTATCGCG |
| 32 | CGCCATATGGAGAGAGCGTGCGCGCTCATGAAATACCTATTGCCTACG |
| 33 | |
| 34 | |
| 35 | |
| 36 | |
| 37 | CGCCATATGGAGAGAGCGTGTGCGCTCGCGATGAAAAAGACAGCTATCGCG |
| 38 | CGCCATATGGAGAGAGCGTGTGCGCTCGCGATGAAATACCTATTGCCTACG |
| 39 | |
| 40 | |
| 41 | |
| 42 | |
| 43 | CGCCATATGGAGGCGAGAGCGTGTGTGGCGGTGATGAAAAAGACAGCTATCGCG |
| 44 | CGCCATATGGAGGCGAGAGCGTGTGTGGCGGTGATGAAATACCTATTGCCTACG |
| 45 | CGCCATATGGAGAGAGCGTGTGCGCTCGCGATGAAAAAGACAGCTATCGCG |
| 46 | CGCCATATGGAGAGAGCGTGTGCGCTCGCGATGAAATACCTATTGCCTACG |
| 47 | CGCCATATGGACAAAGCGTGCTGTGTGGCGGTGATGAAAAAGACAGCTATCGCG |
| 48 | CGCCATATGGACAAAGCGTGCTGTGTGGCGGTGATGAAATACCTATTGCCTACG |
| 49 | |
| 50 | |
| 51 | CGCCATATGGAGAGAGCGTGCGCGCTCGCGGCGATGAAAAAGACAGCTATCGCG |
| 52 | CGCCATATGGAGAGAGCGTGCGCGCTCGCGGCGATGAAATACCTATTGCCTACG |
| 53 | |
| 54 | |
| 55 | |
| 56 | |
| 57 | |
| 58 | |
| 59 | |
| 60 | |
| 61 | |
| 62 | |
| 63 | |
| 64 | |
| 65 | |
| 66 | |
| 67 | |
| 68 | |
| 69 | |
| 70 | |
| 71 | |
| 72 | |
| 73 | |
| 74 | |
| 75 | |
| 76 | |
| 77 | CGCCATATGATGAAAAAGACAGCTATCGCG |
| 78 | CGCCATATGATGAAATACCTATTGCCTACG |

| | |
|---|---|
| Note: the primer No. in the table corresponds to P or SEQ ID NO. | |

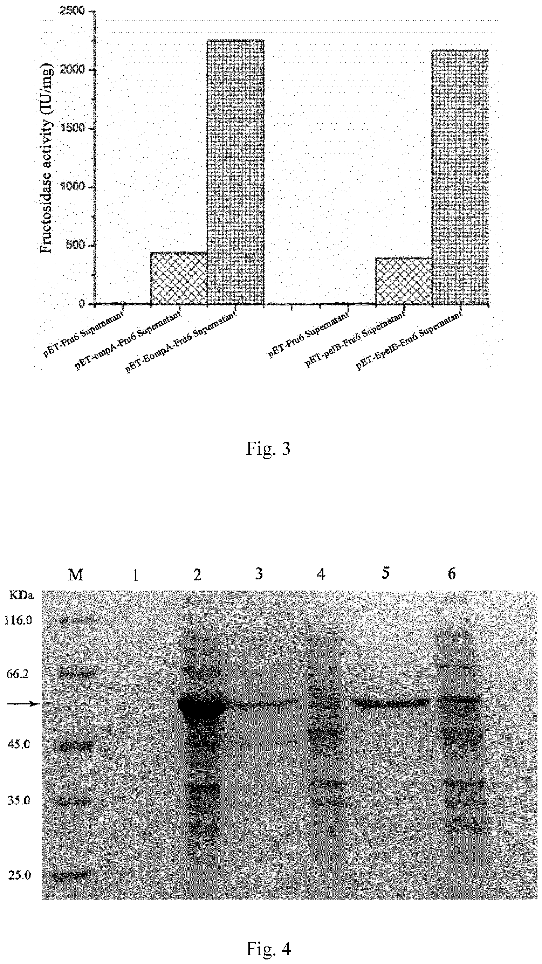

(4) Construction of secretion enhancing expression strain: pET-EompA-FRU6 and pET-EpelB-FRU6 vectors are transformed into the host BL21. See *"Molecular Cloning Manual"* for the operating method. The E. coli BL21 recombinant strains containing the pET-EompA-FRU6 and the pET-EpelB-FRU6 vectors are screened on LB plate containing 100µg/mL ampicillin, and are named as BL21/pET-EompA-FRU6 and as BL21/pET-EpelB-FRU6.
(5) Secretory expression of fructosidase FRU6: the recombinant strain is inoculated to LB liquid culture medium containing 100 µg/mL ampicillin at 37°C, and inoculated to the fresh LB culture medium (containing 100 µg/mL ampicillin) at 37°C as per 2% inoculation amount after being cultured overnight at 200 rpm. Inducer IPTG (final concentration is 1 mmol/L) is added when OD₆₀₀ is 0.6 - 0.9 after being cultured at 200 rpm, and samples are collected every two hours.
(6) Testing on enzyme activity: supernatant is taken from centrifuged cells and acellular fragmentized liquid is taken from disrupted cells after the recombinant strains are fermented in a shake flask. BL21/pET-EompA-FRU6 and BL21/pET-EpelB-FRU6 fermenting supernatants and intracellular enzyme activities are tested, and the recombinant strain without small peptides is taken as a negative control. See Figure 2 (IPTG induction 0-24 hour(s), comparison between secretion efficiencies of supernatant and fructosidase after fermentation of BL21 hosts with small peptides and without small peptides and signal peptides) and Figure 3 (fermentation and sampling by the shake flask for 6 hours in LB culture medium as per 2% inoculation amount) for results. Under unit thallus mass (mg) condition, BL21/pET-EompA-FRU6 or BL21/pET-EpelB-FRU6 is compared with the negative control fermenting supernatant enzyme in activity (see Table 2 for the enhancing effect of more small peptides, each small peptide is fermented 6 hours and sampled for testing of enzyme activity, samples taken in other times are not listed one by one).
   Testing method of enzyme activity is as follows: ① preparation of substrate: adequately dissolve 0.1g puerarin and 2.8g sucrose into 100 mL 0.05 mol/L and pH6 phosphate buffer; ② reaction system: add 50 µL phosphate buffer (0.05 mol/L, pH 6) to 950 substrate, stand at 35°C for reaction 10 min, and then immediately take 100 µL and add to 900 µL methanol for termination of reaction, and take it as blank control, add another 50 µL enzyme liquid to 950 µL substrate, stand at 35°C for reaction 10 min, finally take out 100 µL and add to 900 µL methanol for termination of reaction, take it as a sample and test the sample with HPLC; ③ definition of enzyme activity unit: take the quantity of enzyme required for consumption of 1 µg puerarin at 35°C every minute as an active unit (U).
(7) SDS-PAGE polyacrylamide gel electrophoresis: see *"Molecular Cloning Manual"* for the operating method, and see Figure 4 and Figure 5 for results.

According to the above enzyme activity testing method, it can be seen from table 2 and table 3 that the enzyme activity is obviously enhanced after the small peptides of the present invention in table 2 is fused to the front end of the signal peptide of ompA or pelB adopted for the secretory expression of fructosidase FRU6, it is shown that the small peptides of the present invention have extremely strong secretory expression enhancing abilities.

### Embodiment 2: Use of small peptides for the secretory expression of dextranase

### (1)Construction of the expression vector: small peptides are added before different signal peptides at the N-end of dextranase BGL

Same as in embodiment 1, the fructosidase of embodiment 1 is replaced with the dextranase gene in the embodiment.

Wherein the sequence of dextranase BGL is from *Bacillus subtilis* subsp. *subtilis* 6051-HGW, the sequence No. of GenBank is CP003329.1, and the scope is from 4011849 to 4012490.

The gene segment of dextranase BGL is amplified by PCR reaction with the primers P79 and P80, and prepared to be T vector pMD-T-BGL. Primers P81-P84 or P85-P88 for PCR are overlapped to fuse dextranase BGL and the signal peptide ompA or pelB respectively. R84 and R88 all have *EcoR*□ enzyme cutting sites. The upstream primers P89-P96 are combined with the downstream primers P84 or P88, and different enhancing motifs can be designed and fused before the ompA or pelB signal peptides.

All primers involved in the embodiment are synthesized by the company Invitrogen (see table 4).

**Table 5 Primers required for Embodiment 2**

| Primer | Sequence (5'→3') |
|---|---|
| 79 | CAAACAGGTGGATCGTTTTTT |
| 80 | TTATTTTTTTGTATAGCGCACCCA |
| 81 | ATGAAAAAGACAGCTATCGCG |
| 82 | AAAAAACGATCCACCTGTTTGAGCTTGGGCTACGGT |
| 83 | TACCGTAGCCCAAGCTCAAACAGGTGGATCGTTTTTT |
| 84 | CCGGAATTTTATTTTTTTGTATAGCGCACCCA |
| 85 | ATGAAATACCTATTGCCTACG |
| 86 | AAAAAACGATCCACCTGTTTGAGCCATGGCTGGTTGGGCAGC |
| 87 | CCAACCAGCCATGGCTCAAACAGGTGGATCGTTTTTT |
| 88 | CCGGAATTTTATTTTTTTGTATAGCGCACCCA |
| 89 | CGCCATATGGAACGAGCATGTGTTGCAATGAAAAAGACAGCTATCGCG |
| 90 | CGCCATATGGAACGAGCATGTGTTGCAATGAAATACCTATTGCCTACG |
| 91 | |
| 92 | |
| 93 | |
| 94 | |
| 95 | CGCCATATGATGAAAAAGACAGCTATCGCG |
| 96 | CGCCATATGATGAAATACCTATTGCCTACG |

| | |
|---|---|
| Note: the primer No. in the table corresponds to P or SEQ ID NO. | |

### (2) Secretory expression of dextranase BGL in LB culture medium

The secretory expression vector of the constructed dextranase BGL is transformed into the expression host BL21 (DE3) so as to obtain the recombinant strains which are named BL21/pET-EompA-BGL and BL21/pET-EpelB-BGL. Transformants are screened from LB plates containing 100 g/mL ampicillin, and plasmids are then extracted for validation. See *"Molecular Cloning Manual"* for the detailed method.

The recombinant strain is inoculated to LB liquid culture medium containing 100 µg/mL ampicillin at 37°C, and inoculated to the fresh LB culture medium (containing 100 µg/mL ampicillin) at 37°C as per 2% inoculation amount after being cultured overnight at 200 rpm. Inducer IPTG (final concentration is 1 mmol/L) is added when OD₆₀₀ is 0,6 - 0,9 after being cultured at 200 rpm and collected after being induced for 6 h.

### (3) Testing method of enzyme activity of dextranase

The supernatant is taken from centrifuged cells and acellular fragmentized liquid is taken from disrupted cells after the recombinant strains are fermented in a shaker flask. BL21/pET-EompA-BGL and BL21/pET-EpelB-BGL fermenting supernatants and intracellular enzyme activities are tested, and the recombinant strain without small peptide is taken as a negative control. See Figure 5 (some results are selected, and sampling and testing on enzyme activity are performed after fermentation 6 h) for the enhancing effect of the small peptide.

Testing on enzyme activity by DNS method: take 1.0 mL of 1% (W/V) barley β-glucan (dissolved in pH 6.5, 20 mmol/L Na₂HPO₄-Citrate buffer) as substrate, add 0.5 mL properly diluted enzyme liquid, react at 45°C for 10 min, test the reducing sugar by the DNS method and take the inactivated enzyme as blank control. Definition of enzyme activity unit: the quantity of enzyme required for 1 µmol reducing sugar produced by the substrate every minute is defined as an active unit (U).

**Table 4 Amino acid sequences and enzyme activities of small peptides involved in corresponding dextranase**

| **n** | **Small peptide motif** | **Primer** | **Signal peptide** | **Enzyme activity** |
|---|---|---|---|---|
| 1 | MERACVA | 89 | ompA | 115 |
| | | 90 | pelB | 98 |
| 2 | MERLCVAV+VERACALA | 91 | ompA | 110 |
| | | 92 | pelB | 95 |
| 3 | MERCLATL+VERLCVAV+VERA CALA | 93 | ompA | 134 |
| | | 94 | pelB | 92 |
| 1 | Blank | 95 | ompA | 47 |
| | | 96 | pelB | 58 |

| | | | | |
|---|---|---|---|---|
| Note: the primer No. in the table corresponds to P or SEQ ID NO. | | | | |

(4) See *"Molecular Cloning Manual"* for the detailed operating method for testing expression products of the recombinant strains BL21/pET-EompA-BGL and BL21/pET-EpelB-BGL. See Figure 6 and Figure 7 for results.

## Claims

1. A small peptide **characterized by**:
a) enhancing signal peptide secretion,
b) being fused to the front end of a signal peptide, and
c) having an amino acid sequence MERACVAV.

2. A small peptide according to claim 1, further **characterized by** being suitable for inducible expression.

3. A small peptide according to any one of the previous claims, further **characterized by** being fused to the N-end of an ompA or of a pelB signal peptide.

4. A small peptide according to any one of the previous claims, further **characterized by** being suitable for use in E. coli.

5. A polynucleotide encoding a small peptide according to any one of the previous claims.

6. A recombinant vector comprising a polynucleotide according to claim 5.

7. A recombinant vector according to claim 6, wherein the recombinant vector is a plasmid.

8. A host cell comprising a recombinant vector according to claim 6 or 7, by means of transformation or transfection.

9. The use of the small peptide according to any one of the claims 1-4, for constructing a vector, enhancing the secretion ability of signal peptides to improve the secretory expression of exogenous proteins.

## Patentansprüche

1. Kleines Peptid, **gekennzeichnet durch**
a) Verstärkung der Signalpeptidsekretation,
b) Fusionierung an das vordere Ende eines Signalpeptids, und
c) das Umfassen einer Aminosäuresequenz MERACVAV.

2. Kleines Peptid nach Anspruch 1, ferner **dadurch gekennzeichnet, dass** es für die induzierbare Expression geeignet sind.

3. Kleines Peptid nach einem der vorhergehenden Ansprüche, ferner **gekennzeichnet durch** die Fusionierung an das N-Ende eines ompA oder eines pelB Signalpeptids.

4. Kleines Peptid nach einem der vorhergehenden Ansprüche, ferner **dadurch gekennzeichnet, dass** es für die Verwendung in E.coli geeignet sind.

5. Polynukleotid, das ein kleines Peptid gemäß einem der vorhergehenden Ansprüche kodiert.

6. Rekombinanter Vektor, umfassend ein Polynukleotid nach Anspruch 5.

7. Rekombinanter Vektor nach Anspruch 6, worin der rekombinante Vektor ein Plasmid ist.

8. Wirtszelle, umfassend einen rekombinanten Vektor nach Anspruch 6 oder 7, mittels Transformation oder Transfektion.

9. Verwendung des kleinen Peptids nach einem der Ansprüche 1-4 zum Aufbau eines Vektors, Verbesserung der Sekretionsfähigkeit von Signalpeptiden zur Verbesserung der sekretorischen Expression exogener Proteine.

## Revendications

1. Petit peptide **caractérisé par** :
a) l'amélioration de la sécrétion du peptide signal
b) étant fusionné à l'extrémité frontale d'un peptide signal et
c) présentant une séquence d'acides aminés MERACVAV.

2. Petit peptide selon la revendication 1, **caractérisé en outre par** son adaptation à l'expression inductible.

3. Petit peptide selon l'une quelconque des revendications précédentes, **caractérisé en outre par** sa fusion à une extrémité N d'un peptide signal ompA ou pelB.

4. Petit peptide selon l'une quelconque des revendications précédentes, **caractérisé en outre par** son adaptation à une utilisation dans E. coli.

5. Polynucléotide codant un petit peptide selon l'une quelconque des revendications précédentes.

6. Vecteur recombinant comprenant un polynucléotide selon la revendication 5.

7. Procédé selon la revendication 6, dans lequel le vecteur recombinant est un plasmide.

8. Cellule hôte comprenant un vecteur recombinant selon la revendication 6 ou 7, au moyen de transformation ou de transfection.

9. Utilisation du petit peptide selon l'une quelconque des revendications 1 à 4, destiné à construire un vecteur, à renforcer la capacité de sécrétion des peptides signaux et à améliorer l'expression de sécrétion des protéines exogènes.
